# EUROPÄISCHE PATENTSCHRIFT

(11) **EP 0 743 848 B1**
(45) Veröffentlichungstag und Bekanntmachung des Hinweises auf die Patenterteilung: **08.11.2000**
(21) Anmeldenummer: 95907635.7
(22) Anmeldetag: 01.02.1995
(51) Int. Cl.: A61K 7/13, C09B 51/00

(54) **2-FLUOR-6-NITROANILINE**
2-FLUORO-6-NITROANILINES
2-FLUORO-6-NITROANILINES

(30) Priorität: 10.02.1994 DE 4404198
(43) Veröffentlichungstag der Anmeldung: 27.11.1996
(73) Patentinhaber: Henkel Kommanditgesellschaft auf Aktien, 40191 Düsseldorf (DE)
(72) Erfinder: ROSE, David, D-40723 Hilden (DE); MEINIGKE, Bernd, D-51371 Leverkusen (DE); HÖFFKES, Horst, D-40595 Düsseldorf (DE)
(86) Internationale Anmeldenummer: EP9500358
(87) Internationale Veröffentlichungsnummer: WO9521603

(56) Entgegenhaltungen:
- EP-A- 0 531 958
- EP-A- 0 532 039
- BE-A- 899 399
- DE-A- 4 018 335
- DE-A- 4 240 684
- FR-A- 2 647 443
- GB-A- 2 186 587
- US-A- 5 169 403
- DATABASE WPI Week 8632 Derwent Publications Ltd., London, GB; AN 86-209523 & JP-A-61 143 339 (KYORIN PHARMACEUTICIC LTD)
- SYNTH. COMMUN. (SYNCAV 00397911), Bd. 23, Nr. 3, 1993 Seiten 357 -60, GEE K. R. ET AL. 'fluoroaryl azides'
- JOURNAL OF FLUORINE CHEM., Bd. 42, Nr. 2, 2.Februar 1989 GB, DAHIYAR. ET AL. 'heterocyclic systems...'
- KOGYO KAGAKU ZASSHI, Bd. 70, Nr. 9, 1967 JAPAN, Seiten 1530-2, ISHIKAWA N. ET AL. 'fluorinated nitroanilines'
- JOURNAL OF THE AMERICAN CHEMICAL SOCIETY, Bd. 73, Nr. 1, 24.Januar 1951 Seiten 153-155, FINGER G. ET AL. 'Aromatic fluorine compounds '

## Beschreibung

Gegenstand der Erfindung sind 2-Fluor-6-nitroanilin-Farbstoffe zum Färben von Fasern natürlichen Ursprungs und synthetischen Fasern.

In der Färberei spielen die direktziehenden Farbstoffe eine herausragende Rolle. Im Sinne der vorliegenden Erfindung ist ein direktziehender Farbstoff ein Farbstoff, der aus einem für die jeweils zu erzielende Färbung geeigneten Medium direkt auf die Faser aufzieht.

Es besteht - insbesondere in der Haarfärberei - ständig Bedarf an neuen Farbstoffen, die den hohen an Lichtechtheit, Waschechtheit, Reibechtheit, Farbbrillanz und Aufziehvermögen gestellten Anforderungen entsprechen.

Nitroaromaten, die mit mindestens einem Fluoratom und einer Aminogruppe substituiert sind, z. B. aus den deutschen Offenlegungsschriften DE 37 07 273, DE 39 17 114 und DE 40 18 335 bekannt.

Es wurde nun gefunden, daß ganz bestimmte 2-Fluor-6-nitroaniline die oben genannten an direktziehende Farbstoffe gestellten Anforderungen in besonderem Maße erfüllen.

Gegenstand der Erfindung ist die Verwendung von 2-Fluor-6-nitroanilinen der Formel I 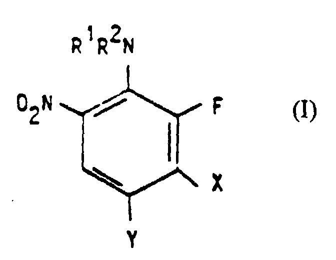 in der R¹ und R² unabhängig voneinander für Wasserstoffe, C₁-C₄-Alkylgruppen, C₂-C₄-Hydroxyalkylgruppen, C₂-C₄-(C₁-C₄-Alkoxy)-alkylgruppen, C₂-C₄-Dihydroxyalkylgruppen, C₂-C₄-Aminoalkylgruppen oder Allylgruppen stehen und NR¹R² auch für einen N-haltigen, gegebenenfalls weitere N-, S- oder O-Atome enthaltenden 5- oder 6-gliedrigen Ring stehen kann, X Wasserstoff, Fluor oder eine Gruppe NR³R⁴ darstellt, in der R³ und R⁴ unabhängig voneinander für Wasserstoffe, C₁-C₄-Alkylgruppen, C₂-C₄-Hydroxyalkylgruppen, C₂-C₄-(C₁-C₄-Alkoxy)-alkylgruppen, C₂-C₄-Halogenalkylgruppen, C₂-C₄-Dihydroxyalkylgruppen, C₂-C₄-Aminoalkylgruppen oder Allylgruppen stehen und NR³R⁴ auch für einen N-haltigen, gegebenenfalls weitere N-, S-oder O-Atome enthaltenden 5- oder 6-gliedrigen Ring stehen kann, und Y Wasserstoff oder Fluor bedeutet, mit der Maßgabe, daß X und Y nicht gleichzeitig Wasserstoffe darstellen, zum Färben von Fasern natürlichen Ursprungs und synthetischen Fasern.

EP-A-0531958 offenbart die Verbindung 3,4-Difluoro-2-(4-methyl-1-piperazinyl)-nitrobenzol als Vorprodukt für die Synthese antibakteriell wirkender 6-Aminochinolone.

Im Journal of the Chemical Society of Japan, Industrial Chemistry Section, September 1967, 70, No. 9, Seiten 1530 bis 1532, werden fluorierte Nitroaniline als Vorstufen zur Herstellung von Azofarbstoffen beschrieben.

EP-A1-0532039 beschreibt N-Trifluoralkyl-Derivate des 2-Nitro-4-aminoanilins als direktziehende Haarfarbstoffe. Substituierte 5-Fluor-2-nitro-p-phenylendiamine und ihre Verwendung zum Färben von Keratinfasern sind Gegenstand der FR-A-2647443. Schließlich offenbart die US-A-5,169,403 quaternierte direktziehende Farbstoffe auf Basis von Nitroanilinen zum Färben von Haaren.

Unter Fasern natürlichen Ursprungs sind menschliche, tierische und pflanzliche Fasern wie z. B. Seide, Baumwolle, Leinen, Jute und Sisal und Keratinfasern wie Haare, Pelze, Wolle oder Federn aber auch regenerierte oder modifizierte Naturfasern wie z. B. Viskose, Nitro- und Acetylcellulose, Alkyl-, Hydroxyalkyl- und Carboxyalkylcellulosen zu verstehen. Aus der Klasse der synthetischen Fasern sind z. B. Polyamid-, Polyester-, Polyacrylnitril- und Polyurethanfasern zu nennen. Das Färben von Textilfasern geschieht vorzugsweise nach dem Aufziehverfahren bei Temperaturen über 90°C, andere für das Färben von Textilfasern übliche Färbeverfahren sind jedoch ebenfalls geeignet. Die 2-Fluor-6-nitroaniline der Formel I eignen sich jedoch besonders gut zum Färben von Keratinfasern, insbesondere menschlichen Haaren.

Besonders geeignet sind 2-Fluor-6-nitroaniline der Formel I, in denen R¹ für eine Methylgruppe, eine 2-(Dimethylamino)ethylgruppe, eine Allylgruppe, eine 2-Methoxyethylgruppe oder eine 2-Hydroxyethylgruppe und R² für Wasserstoff steht.

Auch 2-Fluor-6-nitroaniline der Formel I, in denen NR¹R² für einen Morpholino- oder einen Pyrrolidinoring steht, eignen sich in besonderem Maße.

Einige der 2-Fluor-6-nitroaniline der Formel I sind literaturbekannte Substanzen, andere jedoch sind bisher noch nicht beschrieben worden. Ein weiterer Erfindungsgegenstand sind daher 2-Fluor-6-nitroaniline der Formel I' 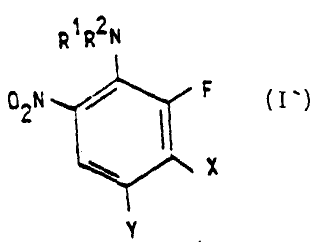 in der R¹ und R² unabhängig voneinander für C₁-C₄-Alkylgruppen, C₂-C₄-Hydroxyalkylgruppen, C₂-C₄-(C₁-C₄-Alkoxy)-alkylgruppen, C₂-C₄-Dihydroxyalkylgruppen, C₂-C₄-Aminoalkylgruppen oder Allylgruppen stehen und NR¹R² auch für einen N-haltigen, gegebenenfalls weitere N-, S- oder O-Atome enthaltenden 5- oder 6-gliedrigen Ring stehen kann,
X Fluor oder eine Gruppe NR³R⁴ darstellt, in der R³ und R⁴ unabhängig voneinander für Wasserstoffe, C₁-C₄-Alkylgruppen, C₂-C₄-Hydroxyalkylgruppen, C₂-C₄-(C₁-C₄-Alkoxy)-alkylgruppen, C₂-C₄-Halogenalkylgruppen, C₂-C₄-Dihydroxyalkylgruppen, C₂-C₄-Aminoalkylgruppen oder Allylgruppen stehen und NR³R⁴ auch für einen N-haltigen, gegebenenfalls weitere N-, S- oder O-Atome enthaltenden 5- oder 6-gliedrigen Ring stehen kann, und Y Wasserstoff bedeutet.

Zur Synthese der neuen 2-Fluor-6-nitroaniline geht man z.B. vom 2,3,4-Trifluornitrobenzol (Handelsprodukt der Firma Hoechst, Deutschland) aus, aus dem durch Umsetzung mit den entsprechenden Aminen unter Fluorwasserstoffabspaltung die 2-Fluor-6-nitroaniline erhalten werden.

Bevorzugt werden die 2-Fluor-6-nitroaniline der Formel I zum Färben von Keratinfasern, insbesondere menschlichen Haaren verwendet, da sie bereits bei physiologisch verträglichen Temperaturen unterhalb 40°C auf Keratinfasern bzw. menschliches Haar aufziehen und dieses intensiv einfärben. Es ist zu beachten, daß in der Haarfärberei an die Farbstoffe und an die erzielten Färbungen besondere Anforderungen gestellt werden. Die Farbstoffe müssen dermatologisch und toxikologisch unbedenklich sein, bei niedrigen Temperaturen auf die Haare aufziehen und gleichzeitig ein gutes Egalisierverhalten zeigen. Die Färbungen müssen gegen Haarbehandlungsmethoden, wie z.B. Dauerwellen, beständig sein. Diesen hohen Anforderungen werden die 2-Fluor-6-nitroaniline der Formel I bzw. die mit ihnen erzielten Färbungen in besonderem Maße gerecht.

Ein weiterer Patentgegenstand sind daher Haarfärbemittel enthaltend 2-Fluor-6-nitroaniline der Formel I in einer Menge von 0,01 bis 5 Gew.-%, vorzugsweise 0,1 bis 2 Gew.-%, bezogen auf das gesamte Haarfärbemittel, und einen wasserhaltigen kosmetischen Träger.

Die Verbindungen der Formel I können dabei sowohl selbst als auch in Form ihrer wasserlöslichen Salze eingesetzt werden. Unter wasserlöslichen Salze sind Salze wie z.B. die Hydrochloride, Hydrobromide oder Sulfate zu verstehen, aber auch Salze, die durch Quaternierung von in den Verbindungen der Formel I enthaltenen Amino-Funktionen, z.B. mit Methyljodid, erhalten werden. Es ist nicht erforderlich, daß eine einheitliche Verbindung der Formel I verwendet wird, vielmehr kann auch eine Mischung verschiedener Verbindungen der Formel I zum Einsatz kommen. Die Farbstoffe der Formel I erzeugen Färbungen in einem weiten Bereich von gelb bis braun, wobei ihr Aufziehvermögen auf die Faser und ihr Egalisierverhalten überdurchschnittlich gut sind. Die erzielten Färbungen zeichnen sich außerdem durch sehr gute Licht-, Schweiß- und Waschechtheit aus.

Wasserhaltige kosmetische Träger sind z.B. Cremes, Emulsionen, Gele oder auch tensidhaltige schäumende Lösungen, z.B. in Shampoos, Schaumaerosolen oder andere Zubereitungen, die für die Anwendung auf dem Haar geeignet sind.

Übliche Bestandteile solcher wasserhaltiger kosmetischer Zubereitungen sind z. B. Netz- und Emulgiermittel, wie anionische, nichtionische und ampholytische Tenside, z. B. Fettalkoholsulfate, Alkansulfonate, α-Ole finsulfonate, Fettalkoholpolyglykolethersulfate, Ethylenoxidanlagerungsprodukte an Fettalkohole, Fettsäure und Alkylphenole, Sorbitanfettsäureester und Fettsäurepartialglyceride, Fettsäurealkanolaminde sowie Verdickungsmittel, wie z. B. Methyl- oder Hydroxycellulose, Stärke, Fettalkohole, Paraffinöle, Fettsäuren, ferner Parfumöle und haarpfleoende Zusätze. wie z. B. wasserlösliche kationische ampholytische und anionische Polymere, Proteinderivate, Pantothensäure und Cholesterin. Die Bestandteile der kosmetischen Träger werden zur Herstellung der erfindungsgemäßen Färbemittel in für diese Zwecke üblichen Mengen eingesetzt, z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Zur Modifikation der Färbungen können den 2-Fluor-6-nitroanilinen der Formel I vorzugsweise weitere übliche direktziehende Farbstoffe, wie z.B. andere Nitrobenzolderivate, Antrachinonfarbstoffe, Triphenylmethan- oder Azofarbstoffe oder aber auch übliche Oxidationshaarfarbstoffvorprodukte zugemischt werden, bei denen zwischen Entwicklerkomponenten und Kupplerkomponenten zu unterscheiden ist. Entwicklerkomponenten bilden durch oxidative Kupplung untereinander oder gegebenenfalls in Gegenwart geeigneter Kupplerkomponenten die Oxidationshaarfarbstoffe aus. Als Entwicklersubstanzen werden z.B. primäre aromatische Amine mit einer weiteren in Para- oder Ortho-Position befindlichen freien oder substituierten Hydroxy- oder Aminogruppe, Diaminopyridinderivate, heterocyclische Hydrazone, 4-Aminopyrazolonderivate und 2,4,5,6-Tetraaminopyrimidin und dessen Derivate eingesetzt. Als Kupplersubstanzen werden Metaphenylendiaminderivate, Naphthole, Resorcin und Resorcinderivate, Pyrazolone und Metaaminophenole verwendet. Weitere direktzierende Farbstoffe und Oxidationshaarfarbstoffvorprodukte können in einer Menge von 0.01 bis 5 Gew.-%, vorzugsweise von 1 bis 3 Gew.-%, bezogen auf das gesamte Haarfärbemittel, enthalten sein.

Die Anwendung der erfindungsgemäßen Haarfärbemittel kann unabhängig von der Art der kosmetischen Zubereitung z. B. als Creme, Gel oder Shampoo im schwach sauren, neutralen oder alkalischen Milieu erfolgen. Bevorzugt ist die Anwendung der Haarfärbemittel in einem pH-Bereich von 6 bis 10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Die nachfolgenden Beispiele sollen den Erfindungsgegenstand näher erläutern, ohne ihn jedoch hierauf zu beschränken.

### Beispiele

### Herstellungsbeispiele

| | | |
|---|---|---|
| 1 | 2,4-Difluor-6-nitroanilin | Handelsprodukt der Fa. Wychem, GB |
| 2 | 2,3-Difluor-6-nitro-N-methylanilin | s. folgende Herstellvorschrift |
| 3 | N-(2,3-Difluor-6-nitrophenyl)-morpholin | s. folgende Herstellvorschrift |
| 4 | N-(2,3-Difluor-6-nitrophenol)-N',N'-dimethylethylendiamin | s. folgende Herstellvorschrift |
| 4a | N,N,N-Trimethyl-2-(5,6-difluor-2-nitroanilino)ethanaminiumjodid | s. folgende Herstellvorschrift |
| 5 | N-(2,3-Difluor-6-nitrophenyl)-allylamin | s. folgende Herstellvorschrift |
| 6 | N-(2,3-Difluor-6-nitrophenyl)-2-methoxyethylamin | s. folgende Herstellvorschrift |
| 7 | 2,6-Bis-(methylamino)-3-nitro-fluorbenzol | s. folgende Herstellvorschrift |
| 8 | 2,6-Bis-(2-hydroxyethylamino)-3-nitrofluorbenzol | s. folgende Herstellvorschrift |
| 9 | 2,6-Bis-(morpholino)-3-nitrofluorbenzol | s. folgende Herstellvorschrift |
| 10 | 2,6-Bis-(pyrrolidino)-3-nitrofluorbenzol | s. folgende Herstellvorschrift |
| 11 | 2,6-Bis-(ethylamino)-3-nitrofluorbenzol | s. folgende Herstellvorschrift |
| 12 | 2-Methylamino-6-ethylamino-3-nitrofluorbenzol | s. folgende Herstellvorschrift |
| 13 | 2-Methylamino-6-N-morpholino-3-nitrofluorbenzol | s. folgende Herstellvorschrift |

2: Eine Mischung bestehend aus 2,3,4-Trifluornitrobenzol (4,4 g) (Fa. Hoechst) und Methylamin (4,1 g in Form einer 40 %igen wäßrigen Lösung) in 25 ml Ethanol wurde 1 Std. bei 20 °C gerührt. Das ausgefallene Produkt wurde abgesaugt und bei 50 °C im Vakuum getrocknet.
   Ausbeute: 4,3 g
   Schmelzpunkt: 89 °C - 93 °C
3: Analog 2, jedoch mit Morpholin anstelle von Methylamin.
   Gelbe Kristalle
   Schmelzpunkt: 141 °C - 145 °C
4: Analog 2, jedoch mit N,N-Dimethylethylendiamin anstelle von Methylamin.
   Gelbe Kristalle
   Schmelzpunkt: 64 °C - 67,5 °C
4a: 1,23 g (5 mMol) der Substanz Nr. 4 wurden in 25 ml Toluol gelöst, bei 25 °C wurden 2,9 g (15 mMol) Methyljodid zugetropft. Anschließend wurde 1 Std. auf dem Wasserbad erhitzt. Nach Abkühlen wurde das Produkt abgesaugt und im Vakuum bei 70 °C getrocknet.
   Gelbe Kristalle
   Schmelzpunkt: 244 °C - 249 °C
5: Analog 2, jedoch mit Allylamin anstelle von Methylamin.
   Gelbes Öl
6: Analog 2, jedoch mit 2-Methoxyethylamin anstelle von Methylamin.
   Gelbe Kristalle
   Schmelzpunkt: 54 °C - 57 °C
7: Eine Mischung bestehend aus 2,3,4-Trifluornitrobenzol (8,8 g) und Methylamin (16,5 g in Form einer 40 %igen wäßrigen Lösung) wurde 3 Std. unter Rückfluß erhitzt. Nach dem Abkühlen wurde das Produkt abgesaugt, mit Wasser nachgewaschen und im Vakuum bei 60 °C getrocknet.
   Orange Kristalle
   Schmelzpunkt: 108 °C - 111 °C
8: Analog 7, jedoch mit Ethanolamin (4 Mol-Äquivalente bezogen auf 2,3,4-Trifluornitrobenzol) anstelle von Methylamin.
   Orange Kristalle
   Schmelzpunkt: 124 °C - 126 °C
9: Analog 7, jedoch mit Morpholin (3 Mol-Äquivalente bezogen auf 2,3,4-Trifluornitrobenzol) anstelle von Methylamin.
   Gelbe Kristalle
   Schmelzpunkt: 79 °C - 82 °C
10: Analog 7, jedoch mit Pyrrolidin (4 Mol-Äquivalente bezogen auf 2,3,4-Trifluornitrobenzol) anstelle von Methylamin.
   Dunkelgelbes Öl
11: Analog 7, jedoch mit Ethylamin (6 Mol-Äquivalente bezogen auf 2,3,4-Trifluornitrobenzol, in Form einer 70 %igen, wäßrigen Lösung) anstelle von Methylamin.
   Orange-Gelbe Kristalle
   Schmelzpunkt: 64 °C - 66 °C
12: 4,45 g Farbstoff 2 wurden bei 20 °C in 30 ml Ethylamin (in Form einer 70 %igen wäßrigen Lösung) eingetragen. Nach 3 Std. Rückfluß wurde nach Abkühlen auf ca. 20 °C das Produkt abgesaugt. Nach Nachwaschen mit Wasser wurde das Produkt im Vakuum bei 60 °C getrocknet.
   Orange Kristalle
   Schmelzpunkt: 88 ° C - 92 °C
13: Analog Farbstoff 12, jedoch mit Morpholin anstelle von Ethylamin.
   Gelbe Kristalle
   Schmelzpunkt: 125 °C - 128 °C

### Anwendungsbeispiele

Färben von Textilfasern:
Zum Färben der Textilfasern wurde ein Mehrfaserbegleitgewebe (Multifiberfabric # 1, Loeffer Textilien, Nettersheim) verwendet. Ein solches Gewebe besteht aus 6 Textilstreifen, bestehend aus Polyacrylnitril, Acetylcellulose (Triacetat), Polyamid, Seide, Viskoseacetat und Wolle.

0,5 g des Farbstoffs (Verbindung 3, 4, 9, 10, 12, 13), 8 g Natriumsulfatdecahydrat und 2 g Natriumcarbonat wurden bei 60 °C in 100 ml Wasser gelöst. Dem Färbebad wurde das Mehrfaserbegleitgewebe zugegeben. Anschliessend wurde innerhalb von 45 Minuten auf eine Temperatur von 98 °C erhöht. Diese Temperatur wurde 1 Stunde lang beibehalten, wobei das verdampfte Wasser kontinuierlich ersetzt wurde. Danach wurde das gefärbte Gewebe zunächst mit kaltem und anschließend mit heißem Wasser gespült. Das Gewebe wurde anschließend 20 Minuten lang in 250 ml 0,25 g Natriumlaurylsulfat enthaltendem Wasser gekocht und anschließend gespült. Zum Schluß wurde das Gewebe getrocknet. Die Färbeergebnisse sind Tabelle 1 zu entnehmen.

**Tabelle 1**

| Fasertyp | 3 | 4 | 9 | 10 | 12 | 13 |
|---|---|---|---|---|---|---|
| Polyacrylnitril | pastellgelb | knallgelb | knallgelb | knallgelb | knallgelb | hellgelb |
| Triacetat | - | - | - | blaßgelb | - | - |
| Polyamid | blaßgelb | hellgelb | blaßgelb | hellgelb | knallgelb | pastellgelb |
| Seide | grauorange | grauorange | korngelb | bernsteingelb | graugelb | grauorange |
| Viskoseacetat | - | - | - | - | - | - |
| Wolle | blaßgelb | gelb | hellgelb | butterblumengelb | pastellgelb | blaßgelb |

### Färben von menschlichen Haaren

Es wurden Haarfärbecremes der folgenden Zusammensetzung hergestellt:

| | |
|---|---|
| Fettalkohol C₁₂₋₁₈ | 10 g |
| Fettalkohol C₁₂₋₁₄ + 2 EO Sulfat, Na-Salz (28 %ig) | 25 g |
| Wasser 60 g | |
| Verbindung 1 - 13 | 1 g |
| Ammoniumsulfat | 1 g |
| konz. Ammioniaklösung | bis pH = 9,5 |
| Wasser | ad 100 g |

Die Bestandteile wurden der Reihe nach miteinander vermischt. Nach Zugabe einer der Verbindungen 1 bis 13 wurde mit konzentrierter Ammoniaklösung der pH-Wert der Emulsion auf 9,5 eingestellt, dann wurde mit Wasser auf 100 g aufgefüllt. Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet. Das Ergebnis der Färbeversuche ist Tabelle 2 zu entnehmen.

**Tabelle 2**

| Verbindung | Nuance des gefärbten Haares |
|---|---|
| 1 | Olivgelb |
| 2 | Orangegelb |
| 3 | Graubraun |
| 4 | Khaki |
| 4a | Honiggelb |
| 5 | Absinthgelb |
| 6 | Olivgelb |
| 7 | Absinthgelb |
| 8 | Honiggelb |
| 9 | Olivbraun |
| 10 | Honiggelb |
| 11 | Honiggelb |
| 12 | Khaki |
| 13 | Olivbraun |

Es wurden Haarfärbecremes, die zusätzlich ein Oxidationshaarfarbstoffvorprodukt enthielten, hergestellt.

### Beispiel 14

| | |
|---|---|
| p-Toluylendiamin | 0,66 g |
| Resorcin | 0,31 g |
| Verbindung 8 | 1,00 g |
| Na₂SO₃ | 1,00 g |
| (NH₄)₂SO₄ | 1,00 g |
| konz. NH₃-Lösung | bis pH = 9,5 |
| Wasser | ad 100 g |

### Beispiel 15

| | |
|---|---|
| p-Amino-o-kresol | 0,37 g |
| 3-Methyl-4-aminophenol | 0,37 g |
| Verbindung 8 | 1,00 g |
| Na₂SO₃ | 1,00 g |
| (NH₄)₂SO₄ | 1,00 g |
| konz. NH₃-Lösung | bis pH = 9,5 |
| Wasser | ad 100 g |

### Beispiel 16

| | |
|---|---|
| p-Amino-o-kresol | 0,37 g |
| p-Toluylendiamin | 0,44 g |
| Verbindung 8 | 0,50 g |
| HC Red Nr. 3 | 0,50 g |
| Na₂SO₃ | 1,00 g |
| (NH₄)₂SO₄ | 1,00 g |
| konz. NH₃-Lösung | bis pH = 9,5 |
| Wasser | ad 100 g |

### Beispiel 17

| | |
|---|---|
| p-Toluylendiamin | 0,22 g |
| 1,3-Bis-(2,4-diaminophenoxy)-propan x 4HCl | 0,45 g |
| Tetraaminopyrimidin | 0,24 g |
| 2-Methylresorcin | 0,12 g |
| Verbindung 8 | 1,00 g |
| Na₂SO₃ | 1,00 g |
| (NH₄)₂SO₄ | 1,00 g |
| konz. NH₃-Lösung | bis pH = 9,5 |
| Wasser | ad 100 g |

### Beispiel 18

| | |
|---|---|
| Verbindung 8 | 0,25 g |
| 1,4-Diaminoanthrachinon | 0,8 g |
| Tris-(2-hydroxyethyl)-2-nitro-1,4-phenylendiamin | 0,1 g |
| Na₂SO₃ | 1,0 g |
| (NH₄)₂SO₄ | 1,0 g |
| konz. NH₃-Lösung | bis pH = 9,5 |
| Wasser | ad 100 g |

### Beispiel 19

| | |
|---|---|
| Verbindung 8 | 0,5 g |
| p-Aminophenol | 0,073 g |
| 5-Amino-2-methylphenol | 0,062 g |
| Na₂SO₃ | 1,0 g |
| (NH₄)₂SO₄ | 1,0 g |
| konz. NH₃-Lösung | bis pH = 9,5 |
| Wasser | ad 100 g |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 9,5 eingestellt, dann wurde mit Wasser auf 100 g aufgefüllt.

Die oxidative Entwicklung der Färbung wurde entweder durch Luftoxidation oder mit Hilfe 3 %iger Wasserstoffperoxidlösung als Oxidationsmittel durchgeführt. Hierzu wurden 100 g der Emulsion mit 50 g Wasserstoffperoxidlösung versetzt und vermischt. Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Die erzielten Ausfärbungen waren folgende:
Beispiel 14 : olivgrün
Beispiel 15 : intensiv rotbraun
Beispiel 16 : violettbraun
Beispiel 17 : dunkelgrün.
Beispiel 18 : bronzebraun
Beispiel 19 : goldbraun

## Patentansprüche

1. Verwendung von 2-Fluor-6-nitroanilinen der Formel I 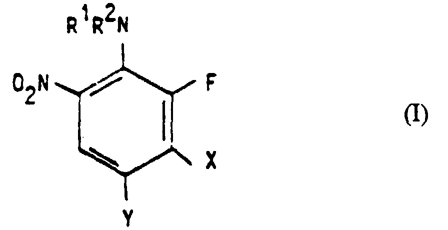 in der R¹ und R² unabhängig voneinander für Wasserstoffe, C₁-C₄-Alkylgruppen, C₂-C₄-Hydroxyalkylgruppen, C₂-C₄-(C₁-C₄-Alkoxy)-alkylgruppen, C₂-C₄-Dihydroxyalkylgruppen, C₂-C₄-Aminoalkylgruppen oder Allylgruppen stehen und NR¹R² auch für einen N-haltigen, gegebenenfalls weitere N-, S- oder O-Atome enthaltenden 5- oder 6-gliedrigen Ring stehen kann,
X Wasserstoff. Fluor oder eine Gruppe NR³R⁴ darstellt, in der R³ und R⁴ unabhängig voneinander für Wasserstoffe, C₁-C₄-Alkylgruppen, C₂-C₄-Halogenalkylgruppen, C₂-C₄-Dihydroxyalkylgruppen, C₂-C₄-Aminoalkylgruppen oder Allylgruppen stehen und NR³R⁴ auch für einen N-haltigen, gegebenenfalls weitere N-, S- oder O-Atome enthaltenden 5- oder 6-gliedrigen Ring stehen kann, und Y Wasserstoff oder Fluor bedeutet, mit der Maßgabe, daß X und Y nicht gleichzeitig Wasserstoffe darstellen, zum Färben von Fasern natürlichen Ursprungs und synthetischen Fasern.

2. Verwendung von 2-Fluor-6-nitroanilinen der Formel I nach Anspruch 1 zum Färben von Keratinfasern, insbesondere menschlichen Haaren.

3. Verwendung nach Anspruch 1 und 2, dadurch gekennzeichnet, daß in der Formel I R¹ für eine Methylgruppe, eine 2-(Dimethylamino)ethylgruppe, eine Allylgruppe, eine 2-Methoxyethylgruppe oder eine 2-Hydroxyethylgruppe und R² für Wasserstoff steht.

4. Verwendung nach Anspruch 1 und 2, dadurch gekennzeichnet daß in der Formel I NR¹R² für einen Morpholino- oder einen Pyrrolidinoring steht.

5. 2-Fluor-6-nitroaniline der Formel I', 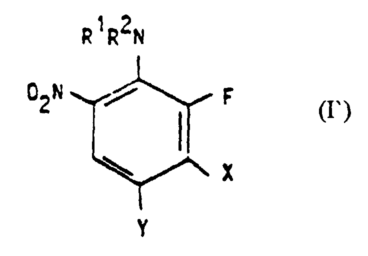 in der R¹ und R² unabhängig voneinander für C₁-C₄-Alkylgruppen, C₂-C₄-Hydroxyalkylgruppen, C₂-C₄-(C₁-C₄-Alkoxy)-alkylgruppen, C₂-C₄-Dihydroxyalkylgruppen, C₂-C₄-Aminoalkylgruppen oder Allylgruppen stehen und NR¹R² auch für einen N-haltigen, gegebenenfalls weitere N-, S- oder O-Atome enthaltenden 5- oder 6-gliedrigen Ring stehen kann,
X Fluor oder eine Gruppe NR³R⁴ darstellt, in der R³ und R⁴ unabhängig voneinander für Wasserstoffe, C₁-C₄-Alkylgruppen, C₂-C₄-Hydroxyalkylgruppen, C₂-C₄-(C₁-C₄-Alkoxy)-alkylgruppen, C₂-C₄-Halogenalkylgruppen, C₂-C₄-Dihydroxyalkylgruppen, C₂-C₄-Aminoalkylgruppen oder Allylgruppen stehen und NR³R⁴ auch für einen N-haltigen, gegebenenfalls weitere N-, S- oder O-Atome enthaltenden 5- oder 6-gliedrigen Ring stehen kann, und Y Wasserstoff bedeutet.

6. Haarfärbemittel enthaltend 2-Fluor-6-nitroaniline der Formel I gemäß Anspruch 1 bis 4 in einer Menge von 0,01 bis 5 Gew.-%, vorzugsweise 0,1 bis 2 Gew.-%, bezogen auf das gesamte Haarfärbemittel, und einen wasserhaltigen kosmetischen Träger.

## Claims

1. The use of 2-fluoro-6-nitroanilines corresponding to formula I: 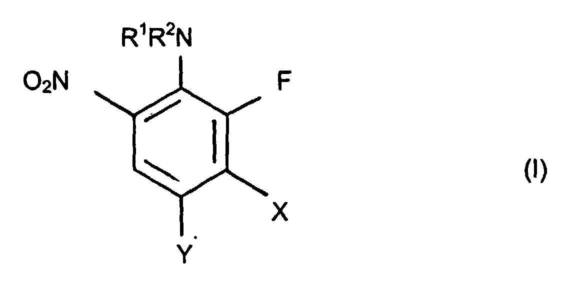 in which R¹ and R² independently of one another represent hydrogen atoms, C₁₋₄ alkyl groups, C₂₋₄ hydroxyalkyl groups, C₂₋₄-(C₁₋₄ alkoxy)-alkyl groups, C₂₋₄ dihydroxyalkyl groups, C₂₋₄ aminoalkyl groups or allyl groups and NR¹R² can also stand for an N-containing 5- or 6-membered ring optionally containing other N, S or O atoms,
X represents hydrogen, fluorine or a group NR³R⁴ where R³ and R⁴ independently of one another represent hydrogen atoms, C₁₋₄ alkyl groups, C₂₋₄ haloalkyl groups, C₂₋₄ dihydroxyalkyl groups, C₂₋₄ aminoalkyl groups or allyl groups and NR³R⁴ can also stand for an N-containing 5- or 6-membered ring optionally containing other N, S or O atoms and
Y represents hydrogen or fluorine, with the proviso that X and Y cannot both be hydrogen atoms,
for colouring fibres of natural original and synthetic fibres.

2. The use of 2-fluoro-6-nitroanilines corresponding to formula I as claimed in claim 1 for colouring keratin fibres, more particularly human hair.

3. The use claimed in claims 1 and 2, characterized in that, in formula I, R¹ represents a methyl group, a 2-(dimethylamino)-ethyl group, an allyl group, a 2-methoxyethyl group or a 2-hydroxyethyl group and R² is hydrogen.

4. The use claimed in claims 1 and 2, characterized in that, in formula I, NR¹R² is a morpholino or pyrrolidino ring.

5. 5-Fluoro-6-nitroanilines corresponding to formula I': 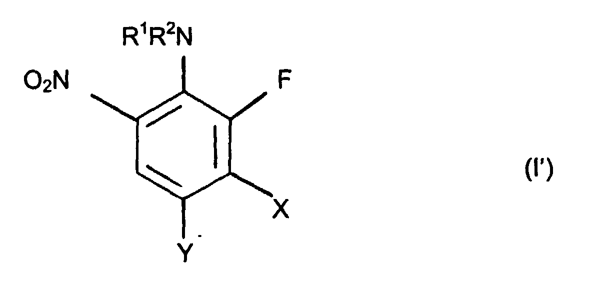 in which R¹ and R² independently of one another represent C₁₋₄ alkyl groups, C₂₋₄ hydroxyalkyl groups, C₂₋₄-(C₁₋₄ alkoxy)-alkyl groups, C₂₋₄ dihydroxyalkyl groups, C₂₋₄ aminoalkyl groups or allyl groups and NR¹R² can also stand for an N-containing 5- or 6-membered ring optionally containing other N, S or O atoms,
X represents fluorine or a group NR³R⁴ where R³ and R⁴ independently of one another represent hydrogen atoms, C₁₋₄ alkyl groups, C₂₋₄ hydroxyalkyl groups, C₂₋₄-(C₁₋₄ alkoxy)-alkyl groups, C₂₋₄ haloalkyl groups, C₂₋₄ dihydroxyalkyl groups, C₂₋₄ aminoalkyl groups or allyl groups and NR³R⁴ can also stand for an N-containing 5- or 6-membered ring optionally containing other N, S or O atoms and
Y represents hydrogen.

6. Hair colorants containing the 2-fluoro-6-nitroanilines of formula I according to claims 1 to 4 in a quantity of 0.01 to 5% by weight and preferably 0.1 to 2% by weight, based on the hair colourant as a whole, and a water-containing cosmetic carrier.

## Revendications

1. Utilisation de 2-fluoro-6-nitroanilines de formule I 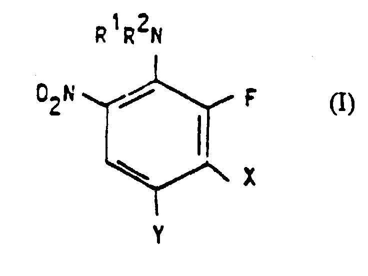 dans laquelle R¹ et R² représentent indépendamment l'un de l'autre des atomes d'hydrogène, des groupes alkyle en C₁ à C₄, des groupes hydroxyalkyle en C₂ à C₄, des groupes (alcoxy en C₁-C₄)-alkyle en C₂ à C₄, des groupes dihydroxyalkyle en C₂ à C₄, des groupes aminoalkyle en C₂ à C₄, ou des groupes allyle, et NR¹R² peut représenter également un noyau à 5 ou 6 chaînons contenant de l'azote, et contenant le cas échéant d'autres atomes de N, S ou O, X représente un hydrogène, un fluor ou un groupe NR³R⁴, dans lequel R³ et R⁴ représentent indépendamment l'un de l'autre des atomes d'hydrogène, des groupes alkyle en C₁ à C₄, des groupes halogénalkyle en C₂ à C₄, des groupes dihydroxyalkyle en C₂ à C₄ , des groupes aminoalkyle en C₂ à C₄, ou des groupes allyle, et NR³R⁴ peuvent également représenter un noyau à 5 ou 6 chaînons contenant de l'azote, et contenant éventuellement d'autres atomes de N, S ou O, et Y représente un hydrogène ou un fluor, sous réserve que X et Y ne représentent pas simultanément des atomes d'hydrogène, pour la coloration de fibres d'origine naturelle et de fibres synthétiques.

2. Utilisation de 2-fluoro-6-nitroanilines de formule I selon la revendication 1 pour la coloration de fibres de kératines en particulier de cheveux humains.

3. Utilisation selon les revendications 1 et 2,
caractérisée en ce que
dans la formule I, R¹ représente un groupe méthyle, un groupe 2-(diméthylamino)éthyle, un groupe allyle, un groupe 2-méthoxyéthyle ou un groupe 2-hydroxyéthyle et R² représente un hydrogène.

4. Utilisation selon la revendication 1 ou 2,
caractérisée en ce que
dans la formule I NR¹R² représente un noyau morpholino ou pyrrolidino.

5. 2-fluoro-6-nitroanilines de formule I' 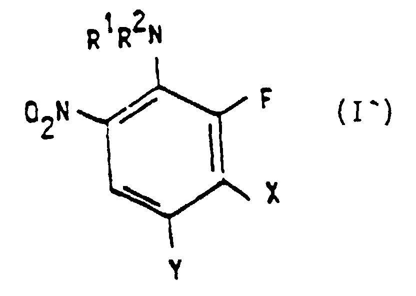 dans laquelle R¹ et R² représentent indépendamment l'un de l'autre des groupes alkyle en C₁ à C₄, des groupes hydroxyalkyle en C₂ à C₄, des groupes (alcoxy en C₁-C₄)-alkyle en C₂ à C₄, des groupes dihydroxyalkyle en C₂ à C₄, des groupes aminoalkyle en C₂ à C₄ ou des groupes allyle, et NR¹R² peuvent également représenter un noyau à 5 ou 6 chaînons contenant de l'azote et contenant le cas échéant d'autres atomes de N, S ou O,
X représente le fluor ou un groupe NR³R⁴, dans lequel R³ et R⁴ représentent indépendamment l'un de l'autre des atomes d'hydrogène, des groupes alkyle an C₁ à C₄, des groupes hydroxyalkyle en C₂ à C₄, des groupes (alcoxy en C₁-C₄)-alkyle en C₂ à C₄, des groupes halogène alkyle en C₂ à C₄, des groupes dihydroxyalkyle en C₂ à C₄, des groupes aminoalkyle en C₂ à C₄ ou des groupes allyle, et NR³R⁴ peut également représenter un noyau à 5 ou 6 chaînons contenant de l'azote et contenant le cas échéant d'autres atomes de N, S ou O, et Y représente un hydrogène.

6. Agent de coloration des cheveux contenant des 2-fluoro-6-nitroanilines de formule I selon les revendications 1 à 4, en une quantité de 0,01 à 5 % en poids, de préférence de 0,1 à 2 % en poids, par rapport à l'ensemble des colorants capillaires, et un support cosmétique contenant de l'eau.
